# EUROPEAN PATENT SPECIFICATION

(11) **EP 1 879 842 B1**
(45) Date of publication and mention of the grant of the patent: **22.08.2012**
(21) Application number: 06744497.6
(22) Date of filing: 13.04.2006
(51) Int. Cl.: C07C 29/62, C07C 31/36

(54) **PROCESS FOR THE PRODUCTION OF ALPHA, GAMMA-DICHLOROHYDRIN FROM GLYCERIN AND HYDROCHLORIC ACID**
VERFAHREN ZUR HERSTELLUNG VON ALPHA,GAMMA-DICHLOROHYDRIN AUS GLYCERIN UND HYDROCHLORIDSÄURE
PROCEDE DE PRODUCTION D'ALPHA, GAMMA-DICHLOROHYDRINE A PARTIR DE GLYCERINE ET D'ACIDE HYDROCHLORIQUE

(30) Priority: 18.04.2005 IT MI20050686
(43) Date of publication of application: 23.01.2008
(73) Proprietor: Eurochem Engineering S.R.L., 20139 Milano (IT)
(72) Inventor: SIANO, Dante, I-20093 Cologno Monzese (IT); SANTACESARIA, Elio, I-20139 Milano (IT); FIANDRA, Valeria, I-80014 Giugliano in Campania (IT); TESSER, Riccardo, I-81100 Caserta (IT); DI NUZZI, Giovanni, 20064 Gorgonzola (IT); DI SERIO, Martino, I-84013 Cava Dei Tirreni (IT); NASTASI, Mario, I-75020 Marconia (IT)
(74) Representative: Modiano, Micaela Nadia
(86) International application number: PCT/IB2006/000875
(87) International publication number: WO 2006/111810

(56) References cited:
- WO-A-2005/054167
- WO-A-2006/020234
- DE-C- 197 308
- DE-C- 197 309
- US-A- 2 144 612
- US-A- 2 198 600

## Description

### Technical field

The present invention relates to a new process for producing α,γ-dichlorohydrin from glycerin and hydrochloric acid, in particular in the presence of catalysts which comprise organic acids.

### Background Art

Among the various possibilities for the use of glycerin in the field of fine chemistry, its use for preparing α,γ-dichlorohydrin is particularly important; α,γ-dichlorohydrin is the intermediate in the process for producing epichlorohydrin, which in turn is used to synthesize epoxy resins [1-7].

The global reaction scheme for preparing α,γ-dichlorohydrin, starting from glycerin and hydrochloric acid, is as follows:

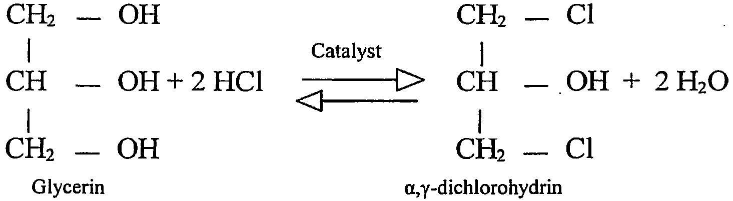

Actually, the reaction proceeds by means of a first chlorination of the glycerin, which mostly forms α-monochlorohydrin and water, with small quantities of β-monochlorohydrin, followed by a second chlorination from which the required product is mainly obtained, i.e., α,γ-dichlorohydrin, and modest quantities of α,β-dichlorohydrin. Accordingly, the more detailed reaction scheme is as follows:

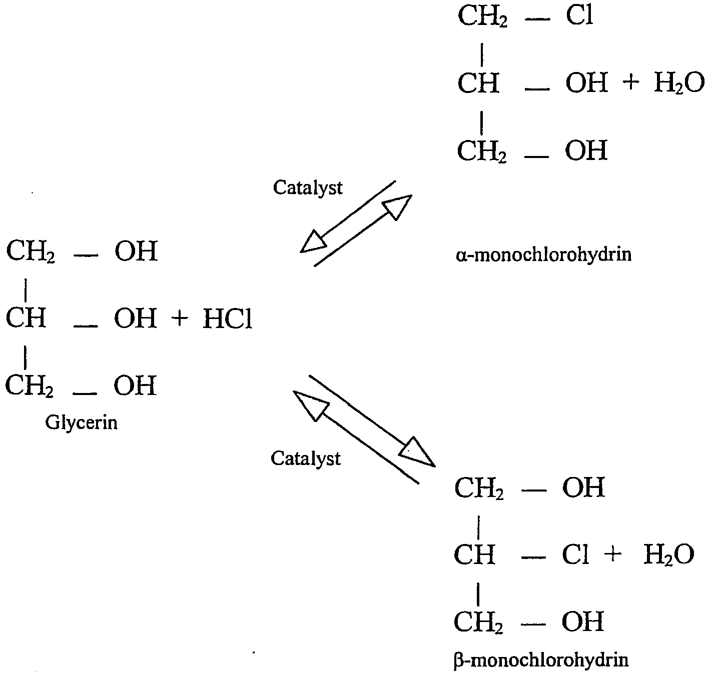

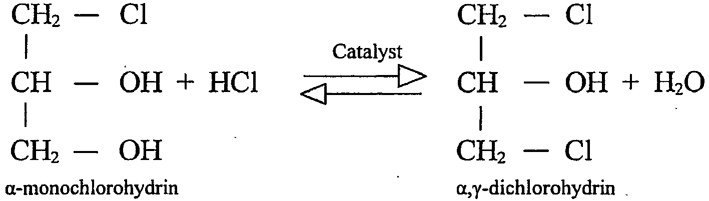

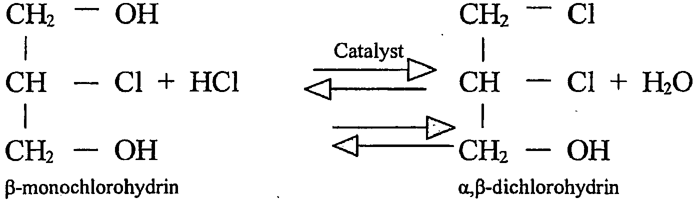

Traditional processes performed starting from glycerin, which besides are rather old, provide for the reaction of the glycerin with hydrochloric acid in solution, in the presence of acetic acid as a catalyst, at the temperature of approximately 80-100°C [8-11].

Some patents describe processes which use an inert organic solvent which is not miscible in water and in which α,γ-dichlorohydrin is soluble, and in which the reaction is performed at the boiling point of the reacting mix, which therefore varies depending on the particular solvent that is used but in any case does not exceed 110°C [12].

Other patents describe the reaction followed by complicated neutralizations, extractions and distillations to recover the α,γ-dichlorohydrin [13, 14].

Traditional processes starting from glycerin therefore suffer from considerable drawbacks, such as:
- the loss of catalyst during the reaction, due to the low boiling point of acetic acid (117°C),
- the slowing of the reaction caused by the introduction of water in the reaction mix, due to the use of aqueous hydrochloric acid and the failure to remove the water that forms as a consequence of the reaction,
- the difficult separation of the α,γ-dichlorohydrin from the reaction mix.

These drawbacks, together with the high cost of glycerin as a raw material, in the past have prevented this process from becoming established. The method currently used employs propylene as a material which yields 30% α,β-dichlorohydrin and 70% α,γ-dichlorohydrin in a mix. Even this process has drawbacks. The high percentage of α,β-dichlorohydrin in fact entails difficulties, since this substance is much slower than α,γ-dichlorohydrin in producing epichlorohydrin in subsequent dehydrochlorination. This has repercussions on the sizing of plants and reduces the yield, forming byproducts [5, 6]. The reaction starting from glycerin would be potentially more advantageous, if the costs of glycerin were reduced. In this regard, it must be noted that the increasing production of biodiesel, which generates glycerin as a byproduct, is causing in general an increasing availability of glycerin and therefore a progressive reduction of its costs. Further, since one of the highest costs in the production of glycerin is its purification, a process in which the chlorination reaction of the glycerin described earlier can occur directly on the unprocessed glycerin, with a further cost reduction, would be useful.

Known from DE 197 308 C is a process for the preparation of dichlorohydrin by reacting glycerin with hydrochloric acid in the presence of different organic acids, propionic acid and succinic acid being exemplified. DE 197 309 C refers to DE 197 308 C and discloses a process for the preparation of dichlorohydrin by reacting glycerin with hydrochloric acid in the presence of different organic acids.

### Disclosure of the Invention

The aim of the present invention is to eliminate the above-mentioned drawbacks for processes for producing α,γ-dichlorohydrin from glycerin and hydrochloric acid, by providing a process which allows to improve considerably the efficiency and economy of the production of α,γ-dichlorohydrin.

Within this aim, an object of the present invention is to provide a process which allows a substantially total conversion of the glycerin.

Another object of the present invention is to provide a process which occurs in thermodynamically favorable conditions, with a reaction rate and a yield which are increased for this reason.

Another object of the present invention is to eliminate the problems related to the separation and quantitative recovery of α,γ-dichlorohydrin.

A further object of the present invention is to provide a process which avoids losses of catalyst during the reaction, so that its concentration remain substantially constant within the reaction mass.

This aim and these and other objects, which will become better apparent from the following description of the invention, are achieved by a process for producing α,γ-dichlorohydrin which comprises the step of reaction between glycerin and hydrochloric acid in the presence of a catalyst which contains an organic acid with a boiling point of more than 120 °C, and the continuous elimination of water and of the α,γ-dichlorohydrin formed by means of the reaction, said elimination occurring by stripping with gaseous hydrochloric acid, either pure or diluted with inert gases.

### Ways of carrying out the invention

Preferably, the hydrochloric acid that is used is anhydrous hydrochloric acid, and the most preferred is gaseous hydrochloric acid, advantageously anhydrous.

The use of gaseous HCl allows to avoid the preliminary introduction of water in the reaction system by means of the aqueous solution, which would have negative consequences on the reaction balance.

The hydrochloric acid might also be used in the form of a nonaqueous solution.

The process according to the invention can be performed in a semicontinuous reactor, with continuous feeding of hydrochloric acid to the reactor as said acid is used up.

The process can also be performed continuously, with continuous feeding also of the glycerin as it is used up and with continuous elimination of water and α,γ-dichlorohydrin formed as an effect of the reaction.

The elimination of the generated water and the collection of the generated α,γ-dichlorohydrin is performed by stripping with gaseous hydrochloric acid, either pure or diluted with a gas which is inert for the reaction.

The α,γ-dichlorohydin and the water can be subsequently separated by fractional condensation.

The organic acid of the catalyst used in the process according to the present invention can be a monocarboxylic acid with 3-10 carbon atoms or a dicarboxylic or polycarboxylic acid with 2-10 carbon atoms.

Non-limiting examples of carboxylic acids which can be used in the present invention are malonic acid, levulinic acid citric acid, succinic acid and propionic acid.

The reaction for chlorination of the glycerin with hydrochloric acid can be performed in the process according to the present invention at a temperature between 100 and 200 °C, preferably between 100 and 175 °C.

The pressure at which the process according to the present invention is performed can be between 50662 and 506625 Pa, preferably between 101325 and 202650 Pa.

In another embodiment, the present invention relates to a catalyst for chlorination of glycerin with hydrochloric acid in order to produce α,γ-dichlorohydrin, which comprises an organic acid with a boiling point of more than 120 °C.

In a particularly preferred embodiment of the process according to the present invention, the use, in the process according to the invention, of gaseous hydrochloric acid, either pure or diluted with a gas which is inert with respect to the reaction, rather than in an aqueous solution, of catalysts which are less volatile with respect to acetic acid, and of a system for the continuous removal of the reaction water, would allow to improve considerably the efficiency and economy of the process.

Use of gaseous HCl instead of HCl in an aqueous solution allows to avoid the preliminary introduction of water in the reaction system by means of the aqueous solution, which would have negative consequences on the reaction balance.

The use of catalysts based on carboxylic acids according to the invention, as an alternative to acetic acid and much less volatile, allows to compensate for the losses of catalyst that occur due to the reaction temperature, which is close to the boiling point of acetic acid, and therefore to keep its concentration constant during the reaction, especially if said reaction occurs in a stream of gaseous HCl, either pure or diluted with a gas which is inert with respect to the reaction.

Further, the use of scarcely volatile organic acids in the present invention allows to perform the reaction at temperatures which are higher than those used in traditional processes and thus increase further the rate of the reaction.

The supply of gaseous HCl into the system can be performed with various systems in order to increase as much as possible the contact area. The α,γ-dichlorohydrin can be recovered, together with the reaction water, by performing for example the reaction in a continuous stream of HCl, either pure or diluted with a gas which is inert with respect to the reaction, with stripping, since α,γ-dichlorohydrin is much more volatile than glycerin and monochlorohydrins.

The process for producing α,γ-dichlorohydrin according to the present invention is performed starting from pure or unprocessed glycerin as obtained as a byproduct in the process for producing biodiesel, with gaseous hydrochloric acid, in the presence of various organic acids, for example monocarboxylic acids with 3 to 10 carbon atoms or di- or tricarboxylic acids with a number of carbon atoms between 2 and 10, as catalysts. In order to ensure a large interfacing surface between the reagents, it is possible to use mechanical agitation, but any method for increasing the interface area has a positive effect especially in the first step of the reaction, which is dominated by the mass transfer. At the laboratory level and in the examples shown herein, the gaseous HCl was made to bubble through in the reaction system through a diffuser. The reaction temperature can be kept between 80 and 180 °C, preferably between 80 and 150 °C. The pressure of the HCl has a positive effect on the reaction rate, but the effect is negative on the stripping of the products; accordingly, a reasonable compromise is to operate in a range from atmospheric pressure to 507000 Pa (5 atm). The reaction can be performed advantageously in a continuous stream of hydrochloric acid with stripping of the generated α,γ-dichlorohydrin and of the reaction water.

The organic acids used as catalysts have a boiling point which is high enough to be able to ignore the losses that occur due to entrainment by the stream of HCl and in any case much higher than the temperature at which the reaction is normally performed.

It is further possible to work easily continuously, by feeding fresh glycerin to the reactor and removing the products of the stripping, α,γ-dichlorohydrin and water, and keeping constant the level of the liquid in the reactor.

The examples given hereinafter describe in detail the tests for producing α,γ-dichlorohydrin performed with various catalysts and in various manners, with reference to the figures.

### Brief description of the drawings

Figure 1 is a view of an experimental apparatus used to perform the experiments cited in the examples;
Figure 2 relates to the reaction in a stream with stripping, with T = 100 °C, comparison between acetic acid and malonic acid (Example 1);
Figure 3 plots the variation of the composition of the reaction mix over time for the reaction under reflux at 110 °C of Example 3;
Figure 4 plots over time the composition of the reaction mix for the reaction with stripping at 100 °C of Example 4;
Figure 5 plots the reaction with succinic acid with T = 100 °C under reflux of Example 7;
Figure 6 plots the reaction with propionic acid at a temperature T = 100 °C under reflux of Example 8;
Figure 7 plots the variation of the concentration of α,γ-dichlorohydrin for the reaction under reflux at 100 °C of Example 9.

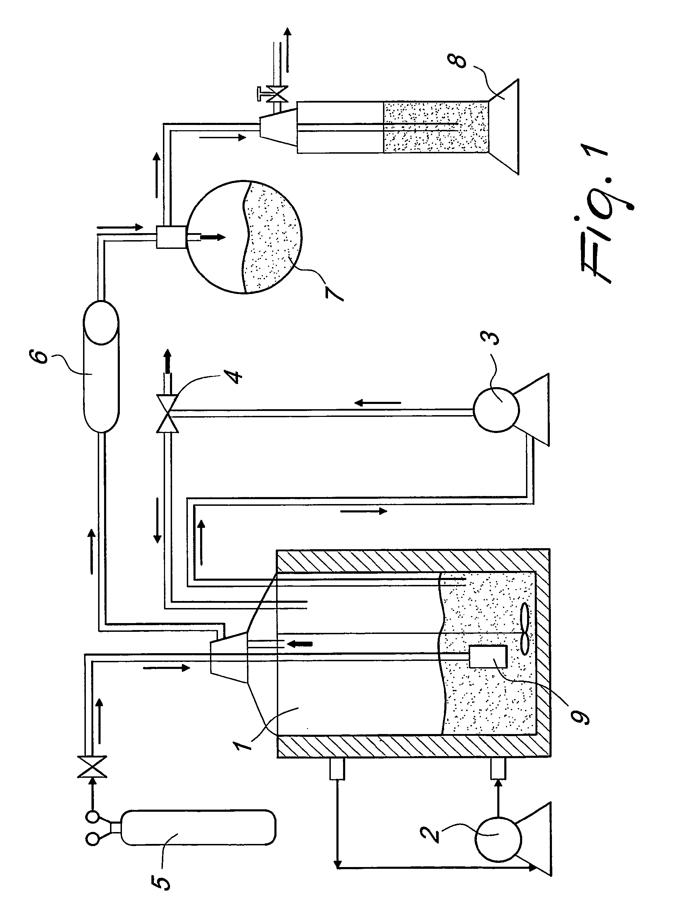

The apparatus used for the tests is shown schematically in Figure 1 and was used both for tests under reflux and for tests with product stripping.

The apparatus comprises:
- a jacketed reactor (1) made of Pyrex glass, with a capacity of approximately 700 ml;
- a thermostat (2) for the circulation of diathermic oil, in order to keep the reaction temperature at the chosen value;
- a peristaltic pump (3), which draws from the bottom of the reactor and returns the reaction mix to the top thereof, making it recirculate within the system;
- a valve (4) for performing the withdrawals from the reaction mix;
- a cylinder (5) of hydrochloric acid;
- a condenser (6) in which cooling water circulates;
- a round-bottomed flask (7) for collecting the reaction product;
- a system (8) for suppressing the excess HCl, constituted by three Drechsel bottles arranged in series, which contain a solution of sodium hydroxide;
- a bubbler (9) provided with a porous partition for introducing gaseous HCl, which ensures effective contact between the gas and the liquid.

In the tests performed with stripping, the excess gas, together with the water and any entrained reaction products, are sent to the condensing system and then to the acidity neutralization system. In the tests performed under reflux, the condenser is instead arranged vertically at the top of the reactor, so that all the reaction products, once cooled, flow back into the reactor and only the excess HCl is sent to the neutralization system.

### Examples

### Example 1

### Reactions in a stream with stripping. Comparison between acetic acid and malonic acid.

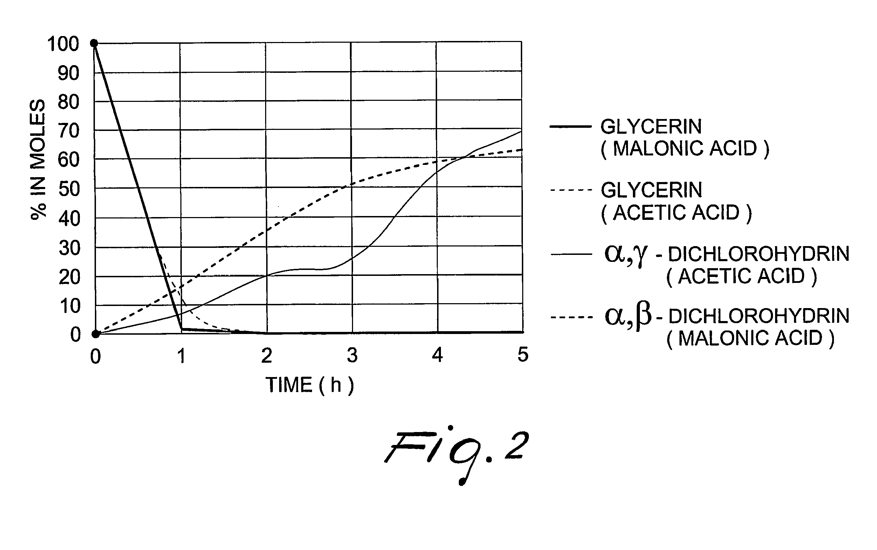

Malonic acid yields excellent results in terms of activity, as clearly shown in Figure 2, which plots, for useful comparison, the concentrations of α,γ-dichlorohydrin and of glycerin as a function of time in two tests, both conducted with stripping, at the temperature of 100 °C. Acetic acid was used in one of these tests and malonic acid was used in the other one. Initially, in both cases, the same amount of catalyst was used, but whereas in the test catalyzed by acetic acid subsequent additions of catalyst were made in order to compensate for the losses caused by stripping by the stream of HCl, in the test in which malonic acid was used the quantity of catalyst remained fixed at the initial value. Since the boiling point of malonic acid is in fact much higher than the reaction temperature (T_{melting-decomp} > 137 °C), it therefore remains in the reactor.

As can be seen, if malonic acid is used, the reaction rate of the glycerin is higher. In one hour, its concentration in fact reaches almost 0, while in the test in which acetic acid is used, two hours are needed for it to disappear completely. Further, the rate of formation of α,γ-dichlorohydrin is also higher in the first four hours of reaction and its concentration is comparable in the two cases only after five hours, since the condition of equilibrium is approached.

What has been described above clarifies the advantage of the use of a catalyst which is less volatile than acetic acid in order to be able to perform the reaction also at higher temperatures, so as to accelerate the reaction rate while avoiding catalyst losses.

### Example 2

### Reactions with malonic acid under reflux at various T values.

The temperature affects the reaction rate considerably, as shown in Table 1, which lists the yields in α,γ-dichlorohydrin after three hours of reaction, in processes performed under reflux at various temperatures by using malonic acid as catalyst. In order to avoid stripping, the output stream of excess HCl was passed through a condenser arranged vertically on the top of the reactor, so that all the products were condensed and flowed back into the reactor.

**Table 1. Reactions with malonic acid, performed under reflux, at various T values.**

| *Yield in a, γ-dichlorohydrin after three hours of reaction.* | | | | |
|---|---|---|---|---|
| T(°C) | 80 | 90 | 100 | 110 |
| 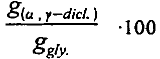 | 22.76% | 35.25% | 55.25% | 57.10% |
| Yield | | | | |

One must bear in mind in any case that the above tests were conducted with the system under reflux, which prevents the removal of the reaction water, and therefore the reaction kinetics is slowed thereby. As will become apparent in the examples given hereafter, in the reaction tests with stripping the conversion of glycerin is substantially complete after only one hour of reaction.

### Example 3

### Reaction with malonic acid under reflux, revolution over time

Operating conditions:
- anhydrous glycerin 99.9% = 200 g
- catalyst = malonic acid, 8% in moles with respect to glycerin
- hydrochloric acid = P ∼ 1.2 bars, flow rate 50 Nl/h
- reaction temperature = 110 °C

This test was conducted by using a bubble condenser connected to the head of the reactor, so that all the reaction products (α-monochlorohydrin, β-monochlorohydrin, α,γ-dichlorohydrin, α,β-dichlorohydrin and water) condensed quantitatively and fell back into the mix. Initially, the reactor was loaded with the glycerin and the catalyst without sending the gaseous HCl.

After a few minutes, the cylinder of HCl was opened and the reaction was started. The formation of monochlorohydrin, predominantly α-monochlorohydrin, is very rapid, and therefore almost all the HCl sent initially reacts and the bubbling of the gas in output from the reactor, in the traps that contain concentrated solutions of NaOH, is minimal.

The reaction was performed for 3 hours, taking samples of the mix every 15 minutes for the first hour and then every 30 minutes for the next 2 hours, in order to perform gas chromatography analyses and thus follow the behavior of the reaction.

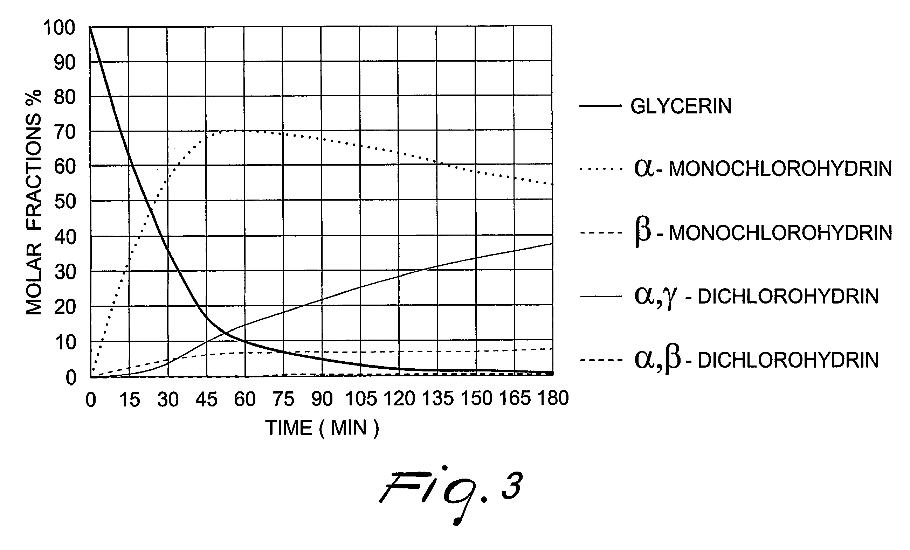

Figure 3 plots the variation of the composition of the reaction mix over time for the reaction performed at this temperature. During the first hour, glycerin decreases rapidly and decays completely in three hours of reaction. The concentration of the α-monochlorohydrin reaches its maximum in 1 hour and then begins to decrease gradually (from 70% to 54% in three hours). The α,γ-dichlorohydrin, after a period of induction of approximately 30 minutes, increases constantly until it reaches 37% in three hours. The formation of α,β-dichlorohydrin remains extremely low (approximately 0.5%) and the concentration of β-monochlorohydrin after 45 minutes of reaction stabilizes at around 6%. The observed behavior highlights the considerable influence of the accumulation of products in the reactor on the kinetic behavior due to the onset of the different chemical balances observed earlier.

### Example 4

### Reaction with the malonic acid, with product stripping

This test again used as its catalyst malonic acid, in a quantity equal to 8% in moles with respect to the glycerin used. However, the reaction was performed with stripping of the products, in the following operating conditions:
- anhydrous glycerin 99.5% = 280 g
- catalyst = malonic acid at 8% in moles with respect to the glycerin
- hydrochloric acid = ΔP ∼ 0.2 bars, flow rate 50 Nl/h
- reaction temperature = 100°C

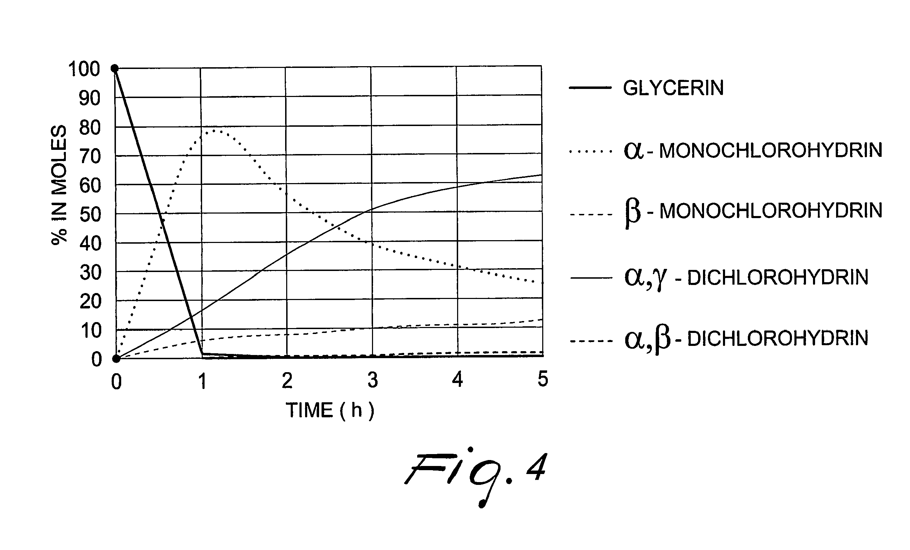

The reactor was loaded with the glycerin and the malonic acid and the system was brought to the reaction temperature of 100 °C. The malonic acid dissolved rather rapidly, under agitation, at 70 °C. The reaction was then started with a stream of HCl which corresponded to a ΔP of approximately 0.2 bars. After 15 minutes, a clear, scarcely viscous product, constituted almost essentially by the water formed during the reaction, began to be collected as an effect of the stripping. The subsequently collected product was found to be gradually more viscous due to removal from the reactor of the α,γ-dichlorohydrin. The reaction was extended for 5 hours, during which samples of the mix were taken hourly. At the end, a sample was also taken from the stripped and condensed product and was analyzed by gas chromatography and by Karl-Fisher analysis, in order to have a complete picture of the behavior of the reaction and of the distribution of the products between the reactor and the distilled fraction. Figure 4 plots the variation over time of the concentrations of the products inside the reactor.

The glycerin, as can be seen, in these conditions is converted completely in approximately one hour and the α-monochlorohydrin reaches the maximum concentration (> 70%) again in one hour of reaction. The increase in α,γ-dichlorohydrin is progressive, while the concentrations of the byproducts remain very low. Karl-Fisher analysis of the water content reveals that less than 1% is present in the reaction mix, while 17% is present in the distilled fraction, confirming that most of the reaction water is stripped by the stream of HCl. After 5 hours, the total concentration of α,γ-dichlorohydrin, i.e., the residual α,γ-dichlorohydrin in the reactor plus the α,γ-dichlorohydrin stripped and collected by condensation, is equal to 76.5% of the theoretical value, the remainder being predominantly α-monochlorohydrin.

### Example 5

### Reaction with levulinic acid under reflux

A test was conducted with levulinic acid as catalyst, in the following operating conditions:
- anhydrous glycerin 99.9% = 201 g
- catalyst = levulinic acid, 8% in moles with respect to the glycerin
- hydrochloric acid = ΔP ∼ 0.2 bars, flow rate 50 Nl/h
- reaction temperature = 100 °C
- test under reflux
- reaction time = 3 hours

The reactor was loaded with the glycerin and the levulinic acid, and the system was brought to the reaction temperature, 100 °C. Then the reaction was started with a stream of HCl equivalent to a ΔP of approximately 0.2 bars.

Gas chromatography analyses show that the α-monochlorohydrin reaches the maximum concentration after 90 minutes of reaction, while the increase in α,γ-dichlorohydrin is progressive, reaching slightly more than 30% in three hours of reaction, and the concentrations of the byproducts remain low.

Table 2 lists the composition of the mix after three hours of reaction.

**Table 2. Reaction with levulinic acid, under reflux at 100 °C**

| Percentage molar fractions after 3 h of reaction | | | | |
|---|---|---|---|---|
| Glycerin | α-monochlorohydrin | β-monochlorohydrin | α,γ-dichlorohydrin | α,β-dichlorohydrin |
| 0.39 | 60.04 | 6.98 | 32.20 | 0.37 |

### Example 6

### Reaction with citric acid under reflux

This test used, as a catalyst, citric acid in the following operating conditions:
- anhydrous glycerin 99.9% = 202 g
- catalyst = citric acid, 8% in moles with respect to the glycerin
- hydrochloric acid = ΔP ∼ 0.2 bars, flow rate 50 Nl/h
- reaction temperature = 100 °C
- test under reflux
- reaction time = 3 hours

Citric acid also was found to be an active catalyst in the process for producing α,γ-dichlorohydrin. In two hours of reaction, the concentration of α-monochlorohydrin reaches its maximum value and the concentration of glycerin decreases significantly. The product of interest increases slowly. However, since the reaction was performed under reflux, it can be easily hypothesized that by removing the water from the system as it forms and by selectively distilling the α,γ-dichlorohydrin, it is possible to accelerate the reaction considerably and achieve good yields.

Table 3 illustrates the composition of the mix after three hours of reaction.

**Table 3. Reaction with citric acid, under reflux at 100 °C**

| Percentage molar fractions after 3 h of reaction | | | | |
|---|---|---|---|---|
| Glycerin | α-monochlorohydrin | β-monochlorohydrin | a,y-dichlorohydrin | α,β-dichlorohydrin |
| 5.15 | 70.45 | 6.49 | 17.61 | 0.26 |

### Example 7

### Reaction with succinic acid, under reflux

The test was conducted in the following operating conditions:
- anhydrous glycerin 99.9% = 200 g
- catalyst = succinic acid, 8% in moles with respect to the glycerin
- hydrochloric acid = ΔP ∼ 0.2 bars, flow rate 50.Nl/h
- reaction temperature = 100 °C
- operating mode under reflux
- reaction time = 150 min

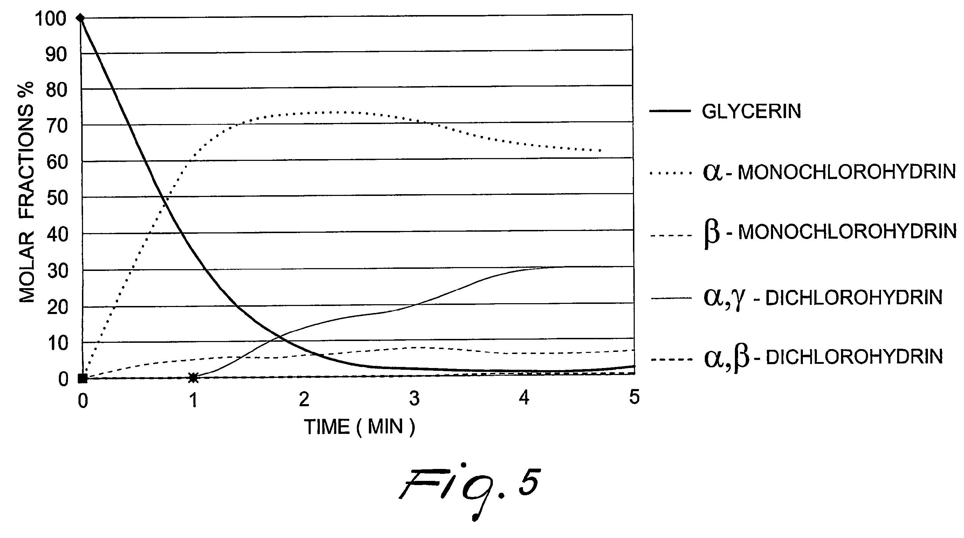

As shown in the chart of Figure 5, succinic acid is an active catalyst in the process for producing α,γ-dichlorohydrin. Glycerin conversion is rather swift, in one hour of reaction the concentration of α-monochlorohydrin reaches its maximum value and the concentration of glycerin decreases significantly. The concentration of the α,γ-dichlorohydrin increases slowly, but since the reaction was conducted under reflux, it can be hypothesized easily that by removing the water from the system as it forms and by stripping selectively the α,γ-dichlorohydrin, it is possible to accelerate considerably the reaction and achieve good yields. The concentration of the γ-monochlorohydrin increases slightly in the initial step but then remains constantly low throughout the reaction. The formation of α,β-dichlorohydrin is negligible.

Table 4 lists the composition of the mix after 150 minutes of reaction.

**Table 4. Reaction with succinic acid, under reflux at T = 100 °C**

| Percentage molar fractions after 150 min of reaction | | | | |
|---|---|---|---|---|
| Glycerin | α-monochlorohydrin | β-monochlorohydrin | α,γ-dichlorohydrin | α,β-dichlorohydrin |
| 1.86 | 60.88 | 6.84 | 30.05 | 0.37 |

### Example 8

### Reaction with propionic acid under reflux

The test with propionic acid as a catalyst was performed in the following operating conditions:
- anhydrous glycerin 99.9% = 200 g
- catalyst = propionic acid, 8% in moles with respect to the glycerin
- hydrochloric acid = ΔP ∼ 0.2 bars, flow rate 50 Nl/h
- reaction temperature = 100 °C
- operating mode under reflux
- reaction time = 3 h

Propionic acid, too, was found to be an active catalyst in the process for producing α,γ-dichlorohydrin.

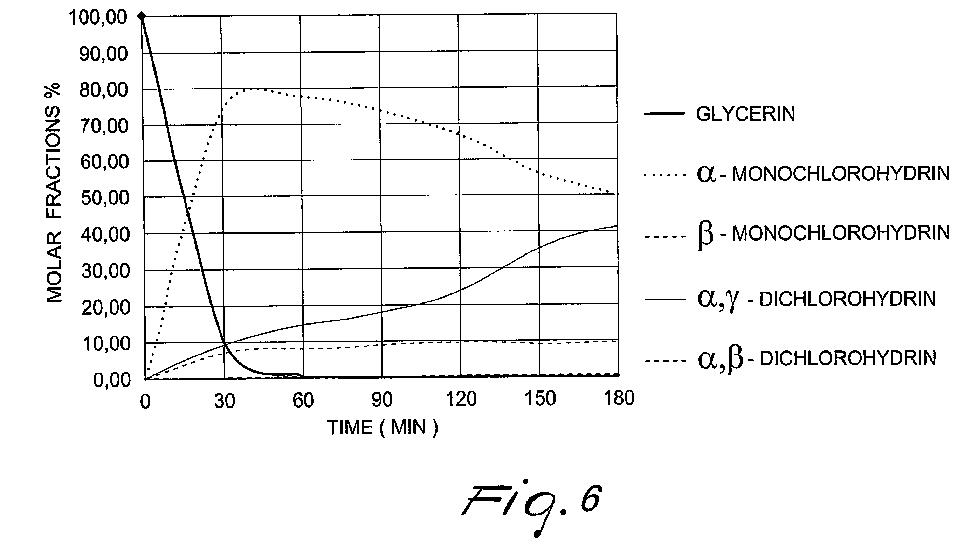

The chart of Figure 6 plots the variation of the composition of the reaction mix over time.

In one hour of reaction, conversion of glycerin is substantially complete and the concentration of the α-monochlorohydrin reaches its maximum value. The formation of α,γ-dichlorohydrin increases gradually over time. In this case also, the catalyst that is used is selective for the formation and the products of interest, since the concentration of the byproducts β-monochlorohydrin and α,β-dichlorohydrin remains low in the reaction mix.

Table 5 plots the composition of the mix after three hours of reaction.

**Table 5. Reaction with_propionic acid, under reflux at T = 100 °C**

| Percentage molar fractions after 3 h of reaction | | | | |
|---|---|---|---|---|
| Glycerin | α-monochlorohydrin | β-monochlorohydrin | α,γ-dichlorohydrin | α,β-dichlorohydrin |
| 0.00 | 49.88 | 8.77 | 41.00 | 0.35 |

### Example 9

### Comparison of activity among the various tested catalysts

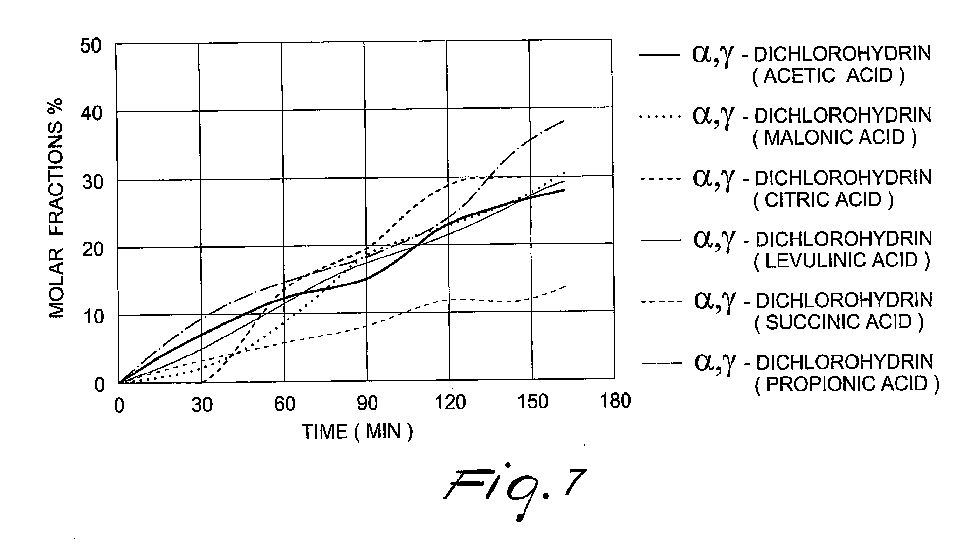

Figure 7 plots the comparison of the kinetic behavior for the various acids used as catalysts in the tests for producing α,γ-dichlorohydrin. In particular, the variation of the concentration of α,γ-dichlorohydrin formed over time is shown in reactions performed at 100 °C under reflux with six different catalysts, such as:
- acetic acid
- malonic acid
- citric acid
- levulinic acid
- succinic acid
- propionic acid

In order to compare the catalytic activity of the various acids, all the reactions were performed under reflux, i.e., so that all the reaction products remained within the reactor. The propionic acid is found to be the best catalyst. Citric acid was found to be slightly less active, but in any case the activity of the various acids is comparable to that of acetic acid, and this confirms that by conducting the reactions at a higher temperature, with stripping of the products, so as to remove the water and α,β-dichlorohydrin, thus shifting to the right the reaction balance, losses of catalyst are avoided, the reaction rate is accelerated, and it is possible to recover easily and selectively the product of interest, which is impure only for water.

From the examples given above, it is therefore evident that the use of catalysts that are less volatile than acetic acid allows to improve considerably the efficiency of the process, which can be performed with stripping, at temperatures higher than 100 °C, avoiding losses of catalyst and recovering selectively the α,γ-dichlorohydrin produced with a high degree of purity. It is further possible to use unprocessed glycerin which arrives for example from the production of biodiesel without subjecting it to expensive refining processes. By working continuously, impurities and byproducts accumulate in the reactor without interfering with the level of purity of the reaction product and by performing a suitable draining without affecting the reaction rate.

### References

[I] GB 799567 (1958)
[2] GB 814695 (1959)
[3] GB 1001364 (1965)
[4] US 4113746(1978)
[5] S. Carrà, E. Santacesaria, and M. Morbidelli, Ind. Eng. Chem. process Des. Dev., Vol. 18, No. 3, part 1, 1979
[6] S. Carrà, E. Santacesaria, and M. Morbidelli, Ind. Eng. Chem. Process Des. Dev., Vol. 18, No. 3, part 2, 1979
[7] Organic Syntheses, CV 1, 233
[8] Organic Syntheses, CV 1, 294
[9] Organic Syntheses, CV 1, 292
[10] Fauconnier, Sanson, Bull. Soc. Chim., 48 (1887), p. 236-240
[1] A.J. Hill and E.J. Fischer, J. Am. Chem. Soc., (1922), Vol. 44, p. 2586-2594
[12] E.C. Britton, R.L. Heindel, US 2,144,612 (1939)
[13] E.C. Britton, H.R. Slagh, US 2,198,600 (1940)
[14] GB 931211 (1963)

## Claims

1. A process for producing α,γ-dichlorohydrin, comprising the step of reaction between glycerin and hydrochloric acid in the presence of a catalyst which contains an organic acid with a boiling point of more than 120 °C and the continuous elimination of water and of the α,γ-dichlorohydrin formed by means of the reaction, said elimination occurring by stripping with gaseous hydrochloric acid, either pure or diluted with inert gases.

2. The process according to claim 1, wherein the hydrochloric acid is gaseous hydrochloric acid, preferably anhydrous hydrochloric acid.

3. The process according to claim 1, wherein the hydrochloric acid is in solution in a nonaqueous solvent.

4. The process according to claim 1, wherein the glycerin is unprocessed glycerin which has not undergone refining processes except for the possible removal of water by evaporation.

5. The process according to any one of the preceding claims, wherein said hydrochloric acid is fed continuously.

6. The process according to claim 5, wherein said glycerin is fed continuously.

7. The process according to claim 1, wherein said α,γ-dichlorohydrin is subsequently separated by fractional condensation.

8. The process according to any one of the preceding claims, wherein said organic acid is a mono-, di- or polycarboxylic acid.

9. The process according to claim 8, wherein said organic acid is selected from the group constituted by malonic acid, levulinic acid, citric acid, succinic acid and propionic acid.

10. The process according to any one of the preceding claims, wherein said reaction is performed at a temperature between 100 and 200 °C, preferably between 100 and 175 °C.

11. The process according to any one of the preceding claims, wherein said reaction occurs at a pressure between 50662 and 506625 Pa, preferably between 101325 and 202650 Pa.

## Patentansprüche

1. Ein Verfahren zur Herstellung von α,γ-Dichlorhydrin, das den Schritt der Reaktion zwischen Glycerin und Salzsäure in Anwesenheit eines Katalysators umfasst, der eine organische Säure mit einem Siedepunkt von über 120°C enthält, und die kontinuierliche Eliminierung von Wasser und dem α,γ-Dichlorhydrin, das durch die Reaktion gebildet wird, wobei die Eliminierung durch Austreibung mit gasförmiger Salzsäure, entweder rein oder mit Edelgasen verdünnt, stattfindet.

2. Das Verfahren gemäß Anspruch 1, worin die Salzsäure gasförmige Salzsäure, vorzugsweise wasserfreie Salzsäure, ist.

3. Das verfahren gemäß Anspruch 1, worin die Salzsäure in Lösung in einem nicht wässerigen Lösungsmittel vorliegt.

4. Das Verfahren gemäß Anspruch 1, worin das Glycerin unverarbeitetes Glycerin ist, das keinen Raffinationsprozessen unterzogen wurde, ausgenommen das mögliche Entfernen von Wasser durch Verdampfung.

5. Das Verfahren gemäß einem beliebigen der obigen Ansprüche, worin die Salzsäure kontinuierlich zugeführt wird.

6. Das Verfahren gemäß Anspruch 5, worin das Glycerin kontinuierlich zugeführt wird.

7. Das Verfahren gemäß Anspruch 1, worin das α,γ-Dichlorhydrin anschließend durch fraktionierte Kondensation abgetrennt wird.

8. Das Verfahren gemäß einem beliebigen der obigen Ansprüche, worin die organische Säure eine Mono-, Di- oder Polycarbonsäure ist.

9. Das Verfahren gemäß Anspruch 8, worin die organische Säure gewählt ist aus der Gruppe bestehend aus Malonsäure, Lävulinsäure, Zitronensäure, Bernsteinsäure und Propionsäure.

10. Das Verfahren gemäß einem beliebigen der obigen Ansprüche, worin die Reaktion bei einer Temperatur zwischen 100 und 200°C, vorzugsweise zwischen 100 und 175°C, durchgeführt wird.

11. Das Verfahren gemäß einem beliebigen der obigen Ansprüche, worin die Reaktion bei einem Druck zwischen 50662 und 506625 Pa, vorzugsweise zwischen 101325 und 202650 Pa, stattfindet.

## Revendications

1. Procédé de production d'α,γ-dichlorohydrine, comprenant l'étape de réaction entre la glycérine et l'acide chlorhydrique en présence d'un catalyseur qui contient un acide organique ayant un point d'ébullition de plus de 120°C et l'élimination continue d'eau et de la α,γ-dichlorohydrine formée par la réaction, ladite élimination se produisant par stripage avec de l'acide chlorhydrique gazeux, soit pur soit dilué avec des gaz inertes.

2. Procédé selon la revendication 1, dans lequel l'acide chlorhydrique est de l'acide chlorhydrique gazeux, de préférence de l'acide chlorhydrique anhydre.

3. Procédé selon la revendication 1, dans lequel l'acide chlorhydrique est en solution dans un solvant non aqueux.

4. Procédé selon la revendication 1, dans lequel la glycérine est de la glycérine non traitée qui n'a pas subi de processus de purification excepté pour l'élimination éventuelle d'eau par évaporation.

5. Procédé selon l'une quelconque des revendications précédentes, dans lequel ledit acide chlorhydrique est fourni de manière continue.

6. Procédé selon la revendication 5, dans lequel ladite glycérine est fournie de manière continue.

7. Procédé selon la revendication 1, dans lequel ladite α,γ-dichlorohydrine est ensuite séparée par condensation fractionnée.

8. Procédé selon l'une quelconque des revendications précédentes, dans lequel ledit acide organique est un acide mono-, di- ou polycarboxylique.

9. Procédé selon la revendication 8, dans lequel ledit acide organique est choisi dans le groupe constitué de l'acide malonique, de l'acide lévulinique, de l'acide citrique, de l'acide succinique et de l'acide propionique.

10. Procédé selon l'une quelconque des revendications précédentes, dans lequel ladite réaction est réalisée à une température entre 100 et 200°C, de préférence entre 100 et 175°C.

11. Procédé selon l'une quelconque des revendications précédentes, dans lequel ladite réaction a lieu à une pression entre 50662 et 506625 Pa, de préférence entre 101325 et 202650 Pa.
